# EUROPEAN PATENT APPLICATION

(11) **EP 4 292 612 A1**
(43) Date of publication of application: **20.12.2023**
(21) Application number: 22752858.5
(22) Date of filing: 14.02.2022
(51) Int. Cl.: A61K 39/395, A61P 19/02, A61P 29/00, A61P 37/06, C07K 16/18, C12N 5/10, C12N 1/15, C12N 1/19, C12N 1/21, C12N 15/13, C12N 15/63, C12P 21/08

(54) **HUMAN DENATURED CRP SPECIFIC NEUTRALIZING ANTIBODY, AND MEDICINE AND ANTI-INFLAMMATORY AGENT CONTAINING SAID ANTIBODY**

(30) Priority: 15.02.2021 JP 2021021509
(71) Applicant: Canon Medical Systems Corporation, Tochigi 324-0036 (JP); General Incorporated Association The Japan Multinational Trial Organization, Nagoya-shi, Aichi 460-0002 (JP)
(72) Inventor: FUJITA, Masaaki, Nagoya-shi, Aichi 460-0002 (JP); HUANG, Cheng-long, Nagoya-shi, Aichi 460-0002 (JP); WADA, Hiromi, Nagoya-shi, Aichi 460-0002 (JP); YASUDA, Yuki, Otawara-shi, Tochigi 324-8550 (JP); SAKURAI, Yasuo, Otawara-shi, Tochigi 324-8550 (JP)
(74) Representative: Marks & Clerk LLP
(86) International application number: PCT/JP2022/005611
(87) International publication number: WO 2022/173036

(57) **Abstract**

The present disclosure relates to a neutralizing antibody specific to human denatured CRP that can be used for a medicine such as an anti-inflammatory agent. More specifically, the present disclosure relates to:
1) an anti-inflammatory agent containing a neutralizing antibody specific to human denatured CRP;
2) a neutralizing antibody specific to human denatured CRP and having an ability of binding to EILFEVPEVT (SEQ ID NO: 2);
3) a polynucleotide comprising a nucleotide sequence encoding a neutralizing antibody specific to human denatured CRP;
4) an expression vector containing the polynucleotide and a promoter operably linked thereto;
5) a transformed cell containing an expression unit containing the polynucleotide and a promoter operably linked thereto; and
6) a medicine containing a neutralizing antibody specific to human denatured CRP and having an ability of binding to EILFEVPEVT (SEQ ID NO: 2).

## Description

### Field

The present disclosure relates to a neutralizing antibody specific to human denatured CRP, and a medicine and an anti-inflammatory agent containing the same.

### Background

C-reactive proteins (CRPs) are generally used as an inflammation marker. It is suggested that there are different types of CRPs in different conformations, that is, native pentameric CRP (pCRP) and a denatured CRP in which constituent factors are dissociated by denaturation of the pentameric CRP, typically monomeric CRP (mCRP), which play different physiological roles [Non Patent Literatures 1 and 2, and Patent Literature 1 (particularly, paragraph [0016])].

Several reports have been made on anti-CRP antibodies. For example, Patent Literature 2 describes an antibody specific to human denatured CRP. The antibody described in Patent Literature 2 does not bind to Peptide 1 (positions 23 to 30), Peptide 2 (positions 109 to 123), or Peptide 3 (positions 137 to 152) of human CRP, and binds to or does not bind to Peptide 4 (positions 199 to 206), and has a property of binding to one of Fragment A (positions 1 to 146) and Fragment B (positions 147 to 206) and not binding to the other (the above positions are understood to be based on the signal removal sequence, considering that the end is set at position 206).

Patent Literature 3 describes an antibody (epitope unknown) having an ability of binding to both normal CRP (that is, native pCRP) and Ca-deletion CRP (denatured CRP).

Patent Literature 4 describes an antibody having an ability of binding to normal CRP (that is, native pCRP). The antibody described in Patent Literature 4 has an epitope at positions 147 to 172 or positions 173 to 206 of human CRP based on the amino acid sequence of SEQ ID NO: 1 in Patent Literature 4 (the amino acid sequence of SEQ ID NO: 1 in Patent Literature 4 is composed of 206 amino acid residues in Full-length).

Patent Literature 5 describes an antibody having an ability of binding to normal CRP (that is, native pCRP). The antibody described in Patent Literature 5 is produced using a peptide antigen selected from the group consisting of Partial peptide 1 (positions 25 to 28), Partial peptide 2 (positions 57 to 60), Partial peptide 3 (positions 114 to 121), Partial peptide 4 (positions 177 to 180), and Partial peptide 5 (positions 204 to 206) of human CRP (the above positions are based on a signal removal sequence).

However, a neutralizing antibody specific to human denatured CRP has not yet been developed.

### Citation List

### Patent Literatures

Patent Literature 1: JP 2007-523837 A
Patent Literature 2: JP H04-505857 A
Patent Literature 3: JP 2004-189665 A
Patent Literature 4: WO 2009/107170 A
Patent Literature 5: JP 2006-115716 A

### Non Patent Literatures

Non Patent Literature 1: Li, H et al., Journal of Biological Chemistry, 2016; 291(16): 8795-8804
Non Patent Literature 2: Wu, Y et al., Biological Chemistry, 2015; 396(11): 1181-1197

### Summary

### Technical Problem

An object of the present disclosure is to provide a neutralizing antibody specific to human denatured CRP which can be used for a medicine such as an anti-inflammatory agent.

### Solution to Problem

As a result of intensive studies, the present inventors have succeeded in producing a neutralizing antibody specific to human denatured CRP having an action of neutralizing human mCRP, and have found that the neutralizing antibody can be used as a medicine such as an anti-inflammatory agent. In particular, a neutralizing antibody specific to human denatured CRP and having an ability of binding to EILFEVPEVT (SEQ ID NO: 2) (corresponding to positions 99 to 108 in the Full-length amino acid sequence of human CRP and positions 81 to 90 in the signal removal sequence) is excellent in the action of neutralizing human mCRP, and thus is promising as a medicine such as an anti-inflammatory agent.

That is, the present disclosure relates to the following and the like.

In one embodiment, provided is an anti-inflammatory agent comprising a neutralizing antibody specific to human denatured CRP.

In a specific embodiment, the above neutralizing antibody may have an ability of binding to EILFEVPEVT (SEQ ID NO: 2).

In a preferred embodiment, the above neutralizing antibody may comprise the following 1) and 2):
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.

In a specific embodiment, the above neutralizing antibody may be IgG.

In a specific embodiment, the anti-inflammatory agent may be for inflammation resulting from an autoimmune disease.

In a preferred embodiment, the above autoimmune disease may be rheumatoid arthritis or systemic lupus erythematosus.

In another embodiment, provided is a neutralizing antibody specific to human denatured CRP and having an ability of binding to EILFEVPEVT (SEQ ID NO: 2).

In a specific embodiment, the above neutralizing antibody may comprise the following 1) and 2):
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.

In still another embodiment, provided is a polynucleotide comprising a nucleotide sequence encoding the above neutralizing antibody.

In still another embodiment, provided is an expression vector comprising the above polynucleotide and a promoter operably linked thereto.

In still another embodiment, provided is a transformed cell comprising an expression unit containing the above polynucleotide and a promoter operably linked thereto.

In still another embodiment, provided is a medicine comprising a neutralizing antibody specific to human denatured CRP and having an ability of binding to EILFEVPEVT (SEQ ID NO: 2).

In a specific embodiment, the above neutralizing antibody may comprise the following 1) and 2):
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.

### Advantageous Effects of Invention

A neutralizing antibody specific to human denatured CRP is useful as an anti-inflammatory agent. The neutralizing antibody is excellent in the action of neutralizing the denatured CRP.

### Brief Description of Drawings

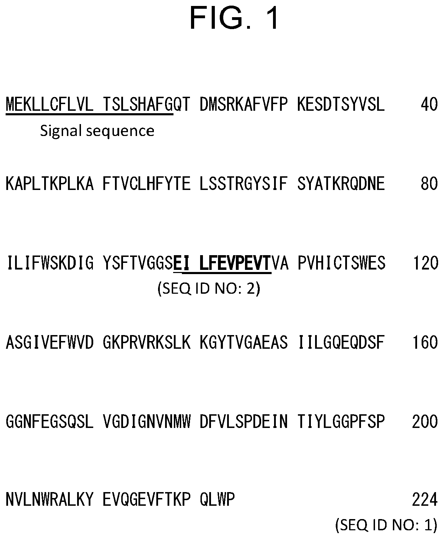

FIG. 1 is a diagram illustrating an amino acid sequence of human CRP (GenBank Accession number: AAL48218), and a neutralizing epitope in human CRP recognized by a neutralizing antibody specific to human denatured CRP (SEQ ID NO: 2) (Positions 99 to 108 in the Full-length amino acid sequence. Positions 81 to 90 in signal removal sequence).

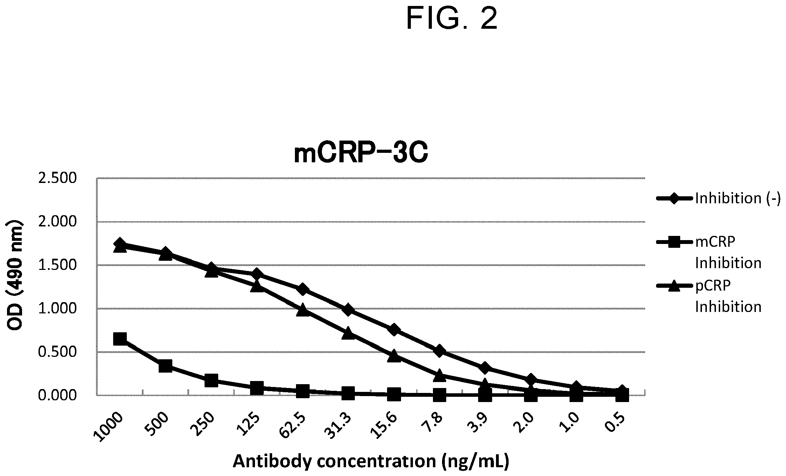

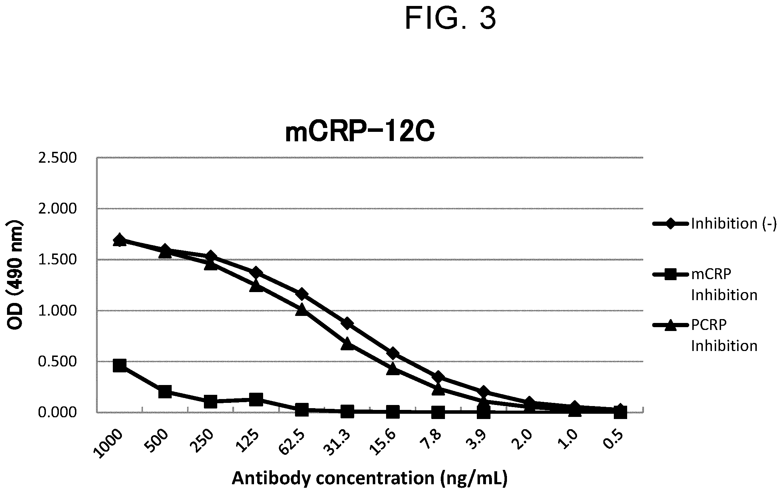

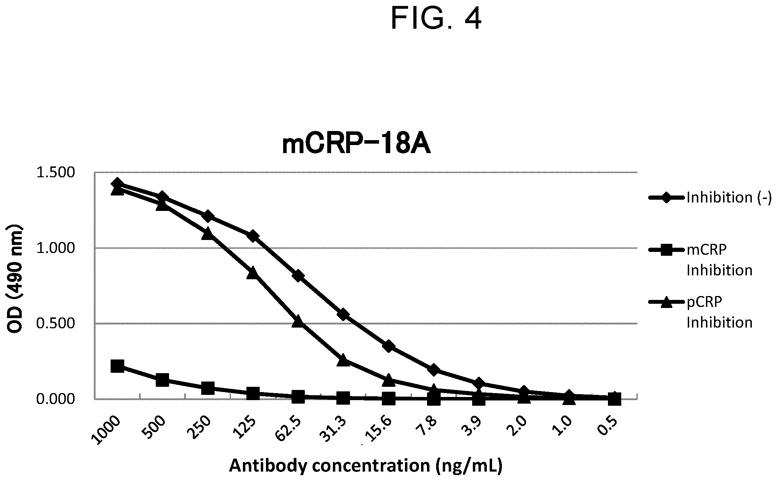

FIG. 2 is a diagram illustrating a result of analyzing the IgG purified from a culture supernatant obtained by culturing hybridoma mCRP-3C by inhibition ELISA. Inhibition (-): an experimental result (control) in the case of using a solution obtained by pre-incubating the IgG purified from the culture supernatant of the hybridoma with a solution not containing both human monomeric CRP (mCRP) and human pentamer (pCRP) (synonymous with simple "CRP"); mCRP inhibition: an experimental result in the case of using a solution obtained by pre-incubating the IgG purified from the culture supernatant of the hybridoma with a solution containing an excess amount of human mCRP; pCRP inhibition: an experimental result in the case of using a solution obtained by pre-incubating the IgG purified from the culture supernatant of the hybridoma with a solution containing an excess amount of human pCRP (the same applies to FIGS. 3 and 4.).
FIG. 3 is a diagram illustrating a result of analyzing the IgG purified from a culture supernatant obtained by culturing hybridoma mCRP-12C by inhibition ELISA.
FIG. 4 is a diagram illustrating a result of analyzing the IgG purified from a culture supernatant obtained by culturing hybridomas mCRP-3C, mCRP-12C, and mCRP-18A by inhibition ELISA.

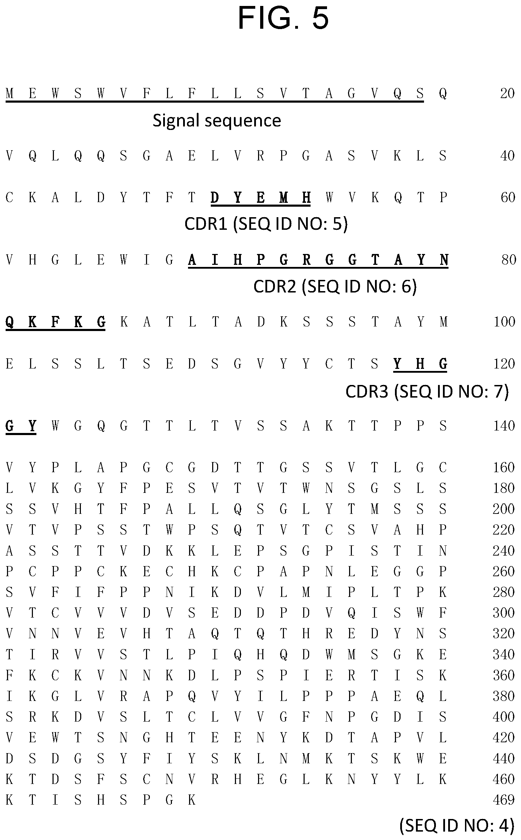

FIG. 5 is a diagram illustrating a signal sequence in a heavy chain of an antibody (3C) purified from a culture supernatant obtained by culturing hybridoma mCRP-3C, and CDR1 (SEQ ID NO: 5), CDR2 (SEQ ID NO: 6) and CDR3 (SEQ ID NO: 7).

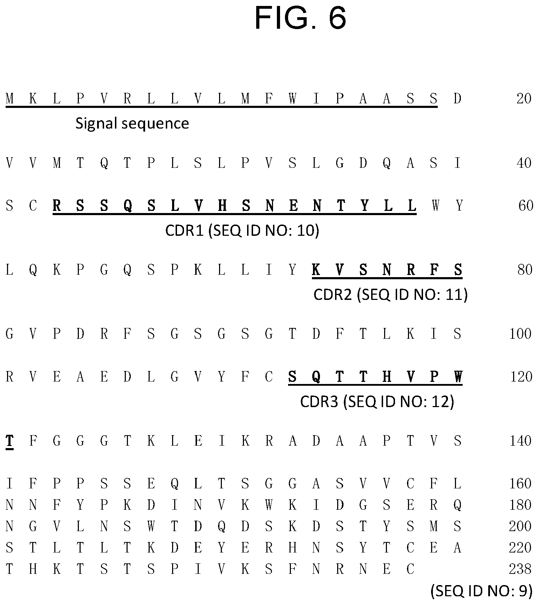

FIG. 6 is a diagram illustrating a signal sequence in a light chain of an antibody (3C) purified from a culture supernatant obtained by culturing hybridoma mCRP-3C, and CDR1 (SEQ ID NO: 10), CDR2 (SEQ ID NO: 11) and CDR3 (SEQ ID NO: 12).

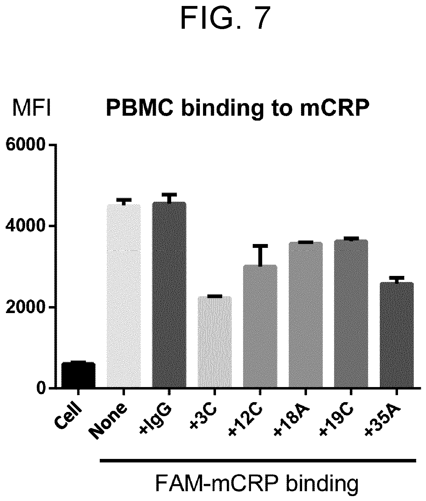

FIG. 7 is a diagram illustrating binding of peripheral blood mononuclear cells (PBMCs) to mCRP.

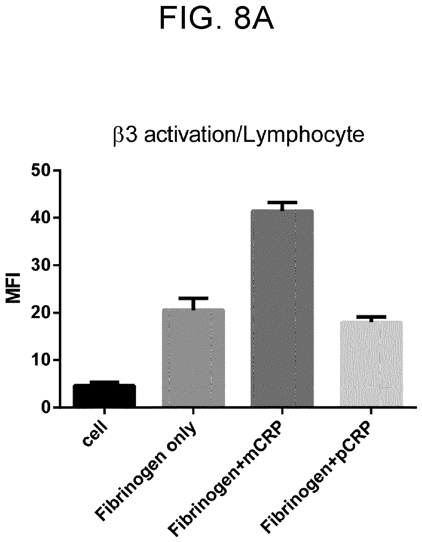

FIG. 8A is a diagram illustrating mCRP-dependent integrin activation (β3 activation/lymphocytes). Lymphocytes have been treated with mCRP and binding of FITC-labeled fibrinogen to the cell surface has been analyzed by flow cytometry. The vertical axis represents the median fluorescence intensity (MFI) (the same applies hereinafter).

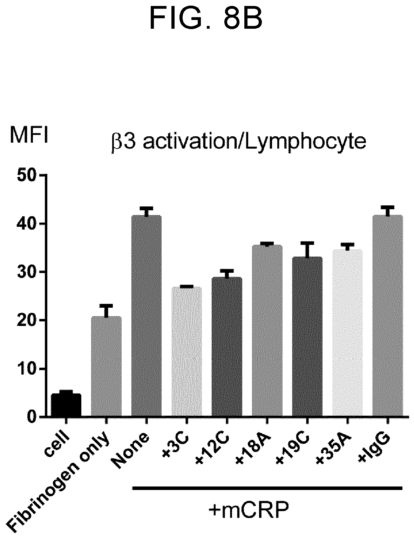

FIG. 8B is a diagram illustrating the inhibition capability of antibodies to mCRP-dependent integrin activation (β3 activation/lymphocytes).

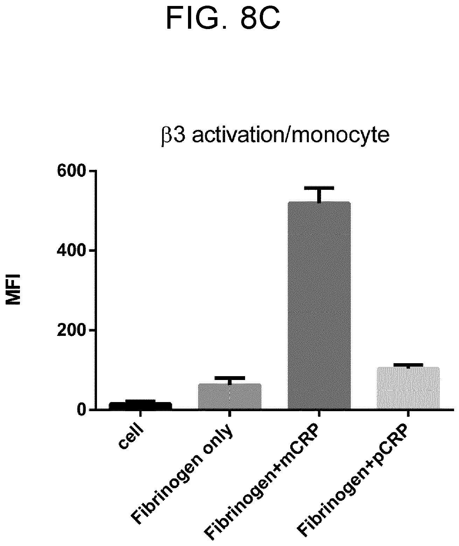

FIG. 8C is a diagram illustrating mCRP-dependent integrin activation (β3 activation/monocytes). Peripheral blood mononuclear cells (PBMCs) have been treated with mCRP and binding of FITC-labeled fibrinogen to the cell surface has been analyzed by flow cytometry.

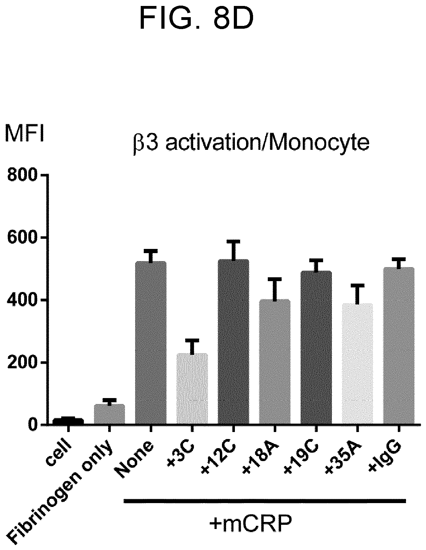

FIG. 8D is a diagram illustrating the inhibition capability of antibodies to mCRP-dependent integrin activation (β3 activation/monocytes).

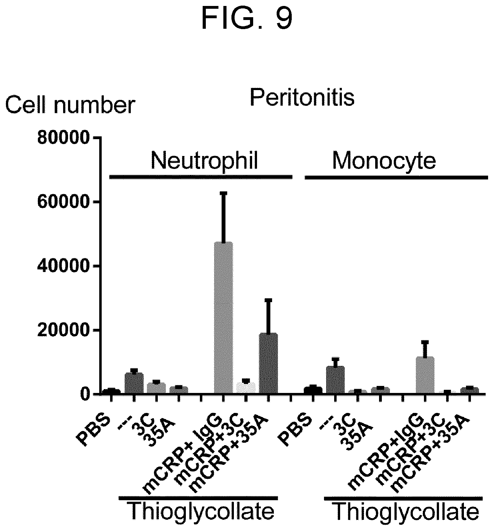

FIG. 9 is a diagram illustrating an inflammation exacerbation effect of mCRP against thioglycollate-induced peritonitis and an inflammation suppressing effect of a neutralizing antibody specific to anti-human denatured CRP. Thioglycolate was intraperitoneally administered to a mouse to induce peritonitis, and the number of neutrophils and monocytes migrated to ascites was measured by flow cytometry. At this time, the influence of the administration of mCRP and neutralizing antibodies (3C and 35A) specific to anti-human denatured CRP on the number of neutrophils and monocytes migrating to ascites was verified (N = 4 or 5).

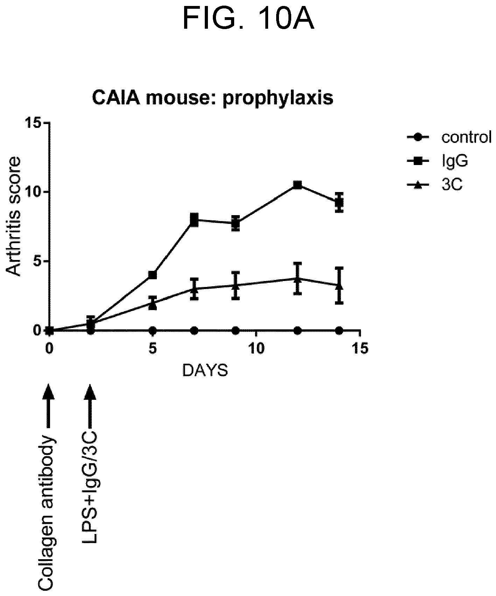

FIG. 10A is a diagram illustrating a preventive effect of an anti-mCRP antibody on anti-collagen antibody-induced arthritis. After 3 days of intravenous administration of four kinds of monoclonal antibodies against type II collagen to mice, arthritis was induced by intraperitoneal administration of lipopolysaccharide (LPS), and the degree of arthritis to the four limbs was measured as Arthritis score. At this time, the influence of the administration of the neutralizing antibody (3C) specific to anti-human denatured CRP on Day 3 on arthritis was verified.

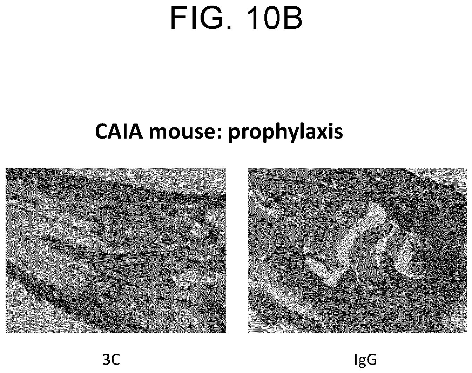

FIG. 10B is a photograph (Day 14) showing the preventive effect of a neutralizing antibody specific to anti-human denatured CRP against anti-collagen antibody-induced arthritis. Mild infiltration of inflammatory cells was observed in the 3C administration group, whereas marked infiltration of inflammatory cells, pannus formation, and destruction of articular cartilage were observed in the IgG administration group.

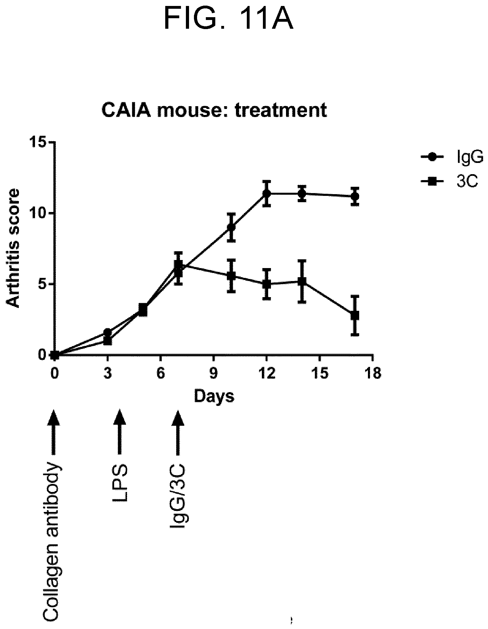

FIG. 11A is a diagram illustrating a therapeutic effect of a neutralizing antibody specific to anti-human denatured CRP against anti-collagen antibody-induced arthritis. After 3 days of intravenous administration of four kinds of monoclonal antibodies against type II collagen to mice, arthritis was induced by intraperitoneal administration of LPS, and the degree of arthritis to the four limbs was measured as Arthritis score. At this time, the influence of the administration of the neutralizing antibody (3C) specific to anti-human denatured CRP on Day 9 on arthritis was verified.

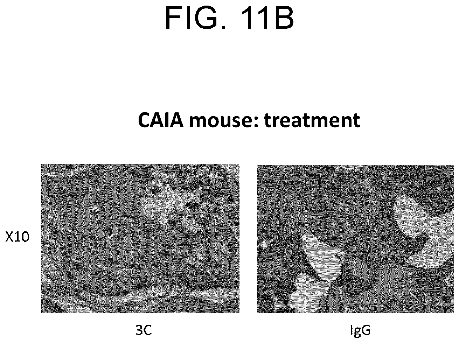

FIG. 11B is a photograph showing a therapeutic effect of a neutralizing antibody specific to anti-human denatured CRP against anti-collagen antibody-induced arthritis. In the 3C administration group, an almost normal bone tissue has been observed, whereas in the IgG administration group, infiltration of inflammatory cells, pannus formation, and destruction of articular cartilage were observed.

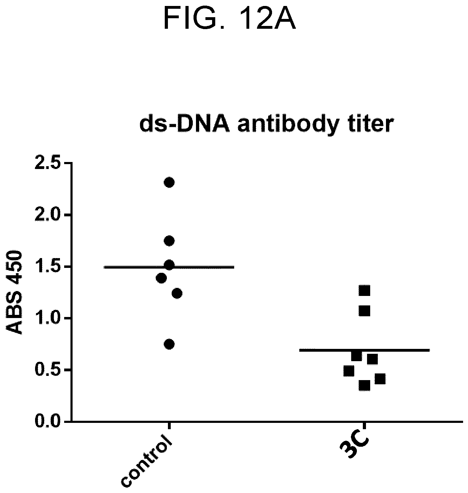

FIG. 12A is a diagram illustrating an inflammation suppressing effect of a neutralizing antibody specific to anti-human denatured CRP on systemic lupus erythematosus (SLE). The neutralizing antibody (3C) specific to human denatured CRP was administered to MRL/lpr mice from weeks 10 to 14, and blood was collected by cardiac puncture at week 18. Anti-double-stranded (ds) DNA antibody titers in blood were measured (N = 7). IgG was used as a control.

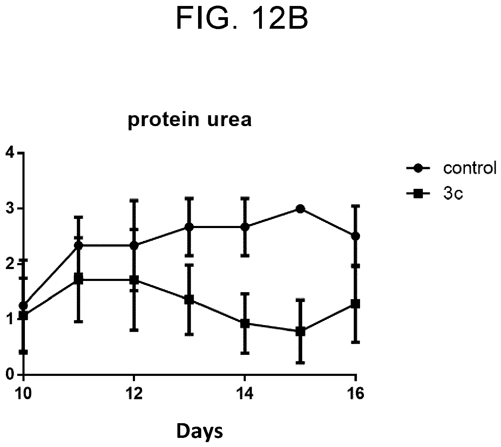

FIG. 12B is a diagram illustrating an inflammation suppressing effect of a neutralizing antibody specific to anti-human denatured CRP on systemic lupus erythematosus (SLE). From week 10, urine protein was measured once a week (N = 7). The horizontal axis represents the number of days, and the error bar indicates the standard deviation.

### Description of Embodiments

The present disclosure relates to an anti-inflammatory agent containing a neutralizing antibody specific to human denatured CRP.

The "human monomeric CRP" (mCRP) refers to a monomeric protein of human CRP (signal sequence may be removed) having the amino acid sequence of SEQ ID NO: 1 (GenBank Accession number: AAL48218) or a naturally occurring variant thereof (for example, natural variant or SNP or haplotype).

The "native CRP" refers to a pentameric protein composed of the human monomeric CRP. Since clinical tests for CRP, which have been widely adopted as indicators of inflammation, have been developed to detect a native CRP, it can be said that the anti-human CRP antibody used in the clinical tests for CRP is an antibody against a native CRP.

The "denatured CRP" refers to a denatured CRP in which constituent factors are dissociated by denaturation of the native CRP. Examples of the denatured CRP include monomeric CRP (mCRP), dimeric CRP, trimeric CRP, and tetrameric CRP, and combinations of one or more kinds (for example, two kinds, three kinds, and four kinds) thereof.

The "human denatured CRP-specific" refers to that an antibody has a higher binding ability to human denatured CRP than to native human pentameric CRP. The antibody specific to human denatured CRP can be obtained by: 1) producing a hybridoma that produces an antibody against human monomeric CRP by using human denatured CRP (preferably, mCRP) as an antigen, 2) selecting a hybridoma that produces an antibody having a higher binding ability for human monomeric CRP than the binding ability for human pentameric CRP by using human denatured CRP and human pentameric CRP as antigens, and 3) isolating the antibody from the culture supernatant of the selected hybridoma, or isolating the antibody from a culture of transformed cells incorporating a gene of the antibody produced by the hybridoma. The antibody specific to the human denatured CRP can be confirmed by checking that the test antibody has a higher binding ability to the human denatured CRP than the binding ability to the human pentameric CRP using the human denatured CRP and the human pentameric CRP as antigens (for example, see inhibition ELISA described in the Examples).

The "neutralizing antibody specific to human denatured CRP" refers to an antibody specific to human denatured CRP and having a neutralizing action on the activity of human denatured CRP (for example, monomeric CRP). It is known that peripheral blood mononuclear cells cannot bind to fibrinogen in the absence of a denatured CRP (for example, mCRP), but fibrinogen can bind to the cell surface because integrin is activated in the presence of a denatured CRP (for example, mCRP). Thus, the neutralizing action on the activity of human denatured CRP (for example, mCRP) may be, for example, suppression of promotion of binding of peripheral blood mononuclear cells to fibrinogen mediated by human denatured CRP (for example, mCRP). The neutralizing action on human denatured CRP (for example, mCRP) can be evaluated, for example, by determining whether the binding of peripheral blood mononuclear cells to fibrinogen in the presence of a denatured CRP (for example, mCRP) and a test antibody is lower than the binding of blood cells (for example, peripheral blood mononuclear cells and lymphocytes) to fibrinogen in the presence of human denatured CRP (for example, mCRP) and in the absence of the test antibody (for example, see Example 5).

In another point of view, a neutralizing antibody specific to human denatured CRP can be said to be an antibody capable of recognizing a neutralizing epitope of human denatured CRP (for example, mCRP). The neutralizing epitope means a partial peptide or partial steric structure of human denatured CRP (for example, mCRP) to which a neutralizing antibody specific to human denatured CRP (for example, mCRP) binds. The neutralizing epitopes can be determined by a method well known in the art.

First, various partial structures of the antigen are produced. In the production of the partial structures, a known oligonucleotide synthesis technique can be used. For example, an epitope can be determined by producing a series of polypeptides sequentially becoming shorter by an appropriate length from the C-terminus or N-terminus of CD147 using genetic recombination techniques well known to those skilled in the art, then examining the reactivity of antibodies against them, determining a rough recognition site, and then synthesizing shorter peptides and examining the reactivity with those peptides. Further, when the antibody does not have a partial peptide but has a partial conformation as an epitope, it is possible to determine which partial conformation the antibody binds to by modifying a specific amino acid sequence to modify the conformation. The epitope that is a partial conformation can also be determined by X-ray structural analysis.

Next, whether or not the epitope is a neutralizing epitope can be determined by evaluating whether or not an antibody capable of recognizing the epitope determined as described above has a neutralizing action on the activity of human denatured CRP (for example, mCRP).

It was confirmed that the 3C antibody and the 35A antibody described in Examples are neutralizing antibodies specific to human denatured CRP (for example, mCRP). Accordingly, by determining the neutralizing epitopes recognized by these antibodies as described above, a neutralizing antibody specific to human denatured CRP can be efficiently produced. For example, a neutralizing antibody specific to human denatured CRP can be obtained by using a hybridoma from a cell collected from an animal (for example, a mouse or a rat) to which a peptide composed of a neutralizing epitope (SEQ ID NO: 2) or a polypeptide rich in the peptide is administered as an antigen. Further, a neutralizing antibody specific to human denatured CRP can be obtained by using a transformed cell into which a gene of an antibody produced by the hybridoma produced as described above is incorporated. Alternatively, a neutralizing antibody specific to human denatured CRP can be obtained by using a peptide composed of a neutralizing epitope (SEQ ID NO: 2) or a polypeptide rich in the neutralizing epitope as an antigen and selecting an antibody having a binding ability to the antigen from an antibody library (for example, human antibody library, humanized antibody library, or human chimeric antibody library).

It is known that there are three complementarity determining regions (CDRs) in each of a heavy chain and a light chain of an antibody molecule. The complementarity determining region, which is also referred to as a hypervariable domain, is a site that is in the variable regions of the heavy chain and the light chain of the antibody and has particularly high variability in the primary structure, and is separated into three portions on the primary structure of the polypeptide chains of the heavy chain and the light chain. These sites are close to each other in conformation and determine specificity to the antigen to which they bind. Accordingly, a neutralizing antibody specific to human denatured CRP can be identified by CDRs.

In certain embodiments, the neutralizing antibody specific to human denatured CRP may be an antibody containing:
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.
In such a neutralizing antibody specific to human denatured CRP, regions of a human antibody may be used as variable regions (for example, a framework region) of a heavy chain and a light chain other than CDRs, and as constant regions of a heavy chain and a light chain.

In a preferred embodiment, the antibody heavy chain containing the variable region comprising CDRs 1 to 3 consisting of the amino acid sequence of the specific SEQ ID NO may be (a) an antibody heavy chain containing an amino acid sequence having an identity of 90% or more to an amino acid sequence consisting of amino acid residues at positions 20 to 133 in the amino acid sequence of SEQ ID NO: 41, SEQ ID NO: 43, SEQ ID NO: 45, SEQ ID NO: 47, SEQ ID NO: 49, or SEQ ID NO: 51 (preferably SEQ ID NO: 43, SEQ ID NO: 45, SEQ ID NO: 47, SEQ ID NO: 49, or SEQ ID NO: 51).

In another preferred embodiment, the antibody light chain containing a variable region comprising CDRs 1 to 3 consisting of the amino acid sequence of the specific SEQ ID NO may be (b) an antibody light chain comprising an amino acid sequence having an identity of 90% or more to an amino acid sequence consisting of amino acid residues at positions 21 to 132 in the amino acid sequence of SEQ ID NO: 53, SEQ ID NO: 55, SEQ ID NO: 57, or SEQ ID NO: 59 (preferably SEQ ID NO: 55, SEQ ID NO: 57 or SEQ ID NO: 59).

In a more preferred embodiment, the neutralizing antibody specific to human denatured CRP may be an antibody containing (a) an antibody heavy chain comprising an amino acid sequence having an identity of 90% or more to an amino acid sequence consisting of amino acid residues at positions 20 to 133 in the amino acid sequence of SEQ ID NO: 41, SEQ ID NO: 43, SEQ ID NO: 45, SEQ ID NO: 47, SEQ ID NO: 49, or SEQ ID NO: 51 (preferably SEQ ID NO: 43, SEQ ID NO: 45, SEQ ID NO: 47, SEQ ID NO: 49, or SEQ ID NO: 51), and (b) an antibody light chain comprising an amino acid sequence having an identity of 90% or more to an amino acid sequence consisting of amino acid residues at positions 21 to 132 in the amino acid sequence of SEQ ID NO: 53, SEQ ID NO: 55, SEQ ID NO: 57, or SEQ ID NO: 59 (preferably SEQ ID NO: 55, SEQ ID NO: 57 or SEQ ID NO: 59).

In a preferred embodiment, the antibody heavy chain containing the variable region comprising CDRs 1 to 3 consisting of the amino acid sequence of the specific SEQ ID NO described above may be (a) an antibody heavy chain comprising an amino acid sequence having an identity of 90% or more to the amino acid sequence of SEQ ID NO: 41, SEQ ID NO: 43, SEQ ID NO: 45, SEQ ID NO: 47, SEQ ID NO: 49, or SEQ ID NO: 51 (preferably SEQ ID NO: 43, SEQ ID NO: 45, SEQ ID NO: 47, SEQ ID NO: 49, or SEQ ID NO: 51).

In another preferred embodiment, the antibody light chain containing a variable region comprising CDRs 1 to 3 consisting of the amino acid sequence of the specific SEQ ID NO described above may be (b) an antibody light chain comprising an amino acid sequence having an identity of 90% or more to the amino acid sequence of SEQ ID NO: 53, SEQ ID NO: 55, SEQ ID NO: 57, or SEQ ID NO: 59 (preferably SEQ ID NO: 55, SEQ ID NO: 57 or SEQ ID NO: 59).

In a more preferred embodiment, the neutralizing antibody specific to human denatured CRP may be an antibody containing (a) an antibody heavy chain comprising an amino acid sequence having an identity of 90% or more to the amino acid sequence of SEQ ID NO: 41, SEQ ID NO: 43, SEQ ID NO: 45, SEQ ID NO: 47, SEQ ID NO: 49, or SEQ ID NO: 51 (preferably SEQ ID NO: 43, SEQ ID NO: 45, SEQ ID NO: 47, SEQ ID NO: 49, or SEQ ID NO: 51) and (b) an antibody light chain comprising an amino acid sequence having an identity of 90% or more to the amino acid sequence of SEQ ID NO: 53, SEQ ID NO: 55, SEQ ID NO: 57, or SEQ ID NO: 59 (preferably SEQ ID NO: 55, SEQ ID NO: 57 or SEQ ID NO: 59).

In another certain embodiment, the neutralizing antibody specific to human denatured CRP may be an antibody containing:
1) an antibody heavy chain containing a variable region comprising an amino acid sequence having an identity of 90% or more to an amino acid sequence consisting of amino acid residues at positions 20 to 133 in the amino acid sequence of SEQ ID NO: 4; and
2) an antibody light chain containing a variable region comprising an amino acid sequence having an identity of 90% or more to an amino acid sequence consisting of amino acid residues at positions 20 to 131 in the amino acid sequence of SEQ ID NO: 9.
In such a neutralizing antibody specific to human denatured CRP, regions of human antibodies may be used as the constant regions of the heavy chain and the light chain.

In yet another certain embodiment, the neutralizing antibody specific to human denatured CRP may be an antibody containing:
1) an antibody heavy chain comprising an amino acid sequence having an identity of 90% or more to an amino acid sequence consisting of amino acid residues at positions 20 to 469 in the amino acid sequence of SEQ ID NO: 4; and
2) an antibody light chain comprising an amino acid sequence having an identity of 90% or more to an amino acid sequence consisting of amino acid residues at positions 20 to 238 in the amino acid sequence of SEQ ID NO: 9.

The identity described above may be 91% or more, 92% or more, 93% or more, 94% or more, 95% or more, 96% or more, 97% or more, 98% or more, or 99% or more. The identity can be calculated by an algorithm blastp. More specifically, the identity can be calculated using Scoring Parameters (Matrix: BLOSUM62; Gap Costs: Existence = 11 Extension = 1; Compositional Adjustments: Conditional compositional score matrix adjustment) set by default in an algorithm blastp provided from National Center for Biotechnology Information (NCBI).

Further, an amino acid sequence having a desired identity to a target amino acid sequence can be identified by mutation of a desired number of amino acid residues to the target amino acid sequence. The mutation of an amino acid residue is one, two, three, or four kinds of mutations selected from the group consisting of deletion, substitution, addition, and insertion of an amino acid residue. The mutation of the amino acid residue may be introduced into one region in the amino acid sequence, or may be introduced into a plurality of different regions. For example, when the target amino acid sequence is composed of 400 or more amino acid residues, an amino acid sequence having an identity of 90% or more to the target amino acid sequence is allowed to have 40 or less amino acid residue mutations to the target amino acid sequence, and an amino acid sequence having an identity of 95% or more to the target amino acid sequence is allowed to have 20 or less amino acid residue mutations to the target amino acid sequence. Further, when the target amino acid sequence is composed of 300 or more amino acid residues, in an amino acid sequence having an identity of 90% or more to the target amino acid sequence, 30 or less amino acid residue mutations to the target amino acid sequence are allowed, and in an amino acid sequence having an identity of 95% or more to the target amino acid sequence, 15 or less amino acid residue mutations to the target amino acid sequence are allowed. Furthermore, when the target amino acid sequence is composed of 200 or more amino acid residues, an amino acid sequence having an identity of 90% or more to the target amino acid sequence is allowed to have 20 or less amino acid residue mutations to the target amino acid sequence, and an amino acid sequence having an identity of 95% or more to the target amino acid sequence is allowed to have 10 or less amino acid residue mutations to the target amino acid sequence. Further, when the target amino acid sequence is composed of 100 or more amino acid residues, an amino acid sequence having an identity of 90% or more to the target amino acid sequence is allowed to have 10 or less amino acid residue mutations to the target amino acid sequence, and an amino acid sequence having an identity of 95% or more to the target amino acid sequence is allowed to have 5 or less amino acid residue mutations to the target amino acid sequence.

The neutralizing antibody specific to human denatured CRP also includes a genetically modified antibody artificially altered for the purpose of reducing heterologous antigenicity to humans, such as a chimeric antibody, a humanized antibody, and a human antibody. Examples of the chimeric antibody include an antibody in which the variable region and the constant region of the antibody are heterologous to each other, for example, a chimeric antibody in which the variable region of a murine- or rat-derived antibody is joined to a human-derived constant region. Examples of the humanized antibody include an antibody in which only CDRs are incorporated into a human-derived antibody, and an antibody in which amino acid residues of some frameworks are also transplanted into a human antibody in addition to the sequence of CDRs by a CDR transplantation method. The human antibody is a human antibody having only a gene sequence of an antibody derived from a human chromosome, and examples thereof include a human antibody obtained by a method using a human antibody-producing mouse having a human chromosome fragment containing genes of a heavy chain and a light chain of a human antibody, and a method using a human antibody library (for example, a method for obtaining a human antibody derived from phage display, and a method for obtaining a human antibody from a human antibody-producing cell).

Further, the neutralizing antibody specific to human denatured CRP may be a single-chain immunoglobulin. Known is a method in which the Full-length sequences of the heavy chain and the light chain of an antibody are linked using an appropriate linker to obtain a single-chain immunoglobulin. By dimerizing such a single-chain immunoglobulin, it is possible to retain a structure and activity similar to those of an antibody that is originally a tetramer. The neutralizing antibody specific to human denatured CRP may be an antibody having a single heavy chain variable region and not having a light chain sequence. Such an antibody is referred to as a single domain antibody (sdAb) or a nanobody, and it has been reported that the antibody retains an ability of binding to an antigen.

Further, it is possible to enhance the antibody-dependent cellular cytotoxicity activity by regulating the sugar chain modification bound to the antibody. Many reports have been made on techniques for regulating a sugar chain modification of an antibody.

When the antibody gene is once isolated and then introduced into an appropriate host to produce an antibody, a combination of an appropriate host and an expression vector can be used. Specific examples of the antibody gene include a combination of a gene encoding a heavy chain sequence and a gene encoding a light chain sequence of the antibody described in the present specification. When transforming the host cell, the heavy chain sequence gene and the light chain sequence gene may be inserted into the same expression vector or may be inserted into separate expression vectors, but can preferably be inserted into the same expression vector. When eukaryotic cells are used as a host, animal cells, plant cells, and eukaryotic microorganisms can be used. Examples of the animal cells include mammalian cells, for example, COS cells which are cells of a monkey, mouse fibroblast NIH3T3, and Chinese hamster ovary cells (CHO cells). When prokaryotic cells are used, examples thereof include E. coli and Bacillus subtilis. These cells are transfected with a target antibody gene by transformation, and the transformed cells are cultured to obtain an antibody. In the above culture method, the yield may vary depending on the sequence of the antibody, and it is possible to select an antibody that is easy to produce as a medicine by using the yield as an index from among antibodies having equivalent binding activity.

Examples of the isotype of the neutralizing antibody specific to human denatured CRP include IgG (for example, IgG1, IgG2, IgG3, and IgG4), IgM, IgA (for example, IgA1 and IgA2), IgD, and IgE, preferably IgG or IgM, and more preferably IgG.

The neutralizing antibody specific to human denatured CRP can be used as an anti-inflammatory agent. For example, the inflammatory disease to which the anti-inflammatory agent may be applied can be classified based on the organ or tissue susceptible to inflammation. Examples of such inflammatory diseases include peritonitis, appendicitis, blepharitis, bronchiolitis or bronchitis (for example, asthma), bursitis, cervicitis, cholangitis, cholecystitis, colitis, conjunctivitis, bladder inflammation, dacryadenitis, dermatitis, dermatomyositis, encephalitis, endocarditis, endometritis, enteritis, epicondylitis, epididymitis, fasciitis, connective tissue inflammation, gastritis, gastroenteritis, hepatitis, laryngitis, mastitis, meningitis, myelitis, myocarditis, myositis, nephritis, oophoritis, orchitis, osteitis, pancreatitis, parotitis, pericarditis, pharyngitis, pleuritis, phlebitis, pneumonia, proctitis, prostatitis, pyelonephritis, otosalpingitis, sinusitis, stomatitis, synovitis, tendinitis, tonsillitis, uveitis, vaginitis, vasculitis, and vulvitis. The inflammatory disease may also be inflammation associated with an infectious disease (for example, viruses, microorganisms, and parasites).

In certain embodiments, the inflammatory disease may be an autoimmune disease. Examples of the autoimmune diseases include arthritis (for example, Rheumatoid arthritis, Psoriatic arthritis, Osteoarthritis, and Juvenile arthritis), systemic lupus erythematosus, adult-onset Still's disease, inflammatory bowel disease, Hashimoto's thyroiditis, Basedow's disease, Sjogren's syndrome, multiple sclerosis, Guillain-Barre syndrome, acute disseminated encephalomyelitis, Addison disease, ankylosing spondylitis, anti-phospholipid antibody syndrome, autoimmune hepatitis, Goodpasture's syndrome, optic neuritis, primary biliary cirrhosis, Reiter's disease, Takayasu arteritis, temporal arteritis, Wegener granulomatosis, psoriasis, alopecia universalis, Behcet's disease, chronic fatigue syndrome, and interstitial cystitis.

In another certain embodiment, the inflammatory disease may be a non-autoimmune disease. Examples of the non-autoimmune diseases include arteriosclerosis, angina pectoris, myocardial infarction, rejection (for example, rejection by transplantation such as organ transplantation), neurodegenerative diseases (for example, Alzheimer's disease), and age-related macular degeneration.

The present disclosure also relates to a neutralizing antibody specific to human denatured CRP and having an ability of binding to EILFEVPEVT (SEQ ID NO: 2) and a medicine containing the same. The details of a neutralizing antibody specific to human denatured CRP and having an ability of binding to EILFEVPEVT (SEQ ID NO: 2) are as described above. Such a medicine is useful for the prevention or treatment of diseases such as the above-mentioned inflammatory diseases.

The present disclosure also relates to a polynucleotide comprising a nucleotide sequence encoding a neutralizing antibody specific to human denatured CRP and having an ability of binding to EILFEVPEVT (SEQ ID NO: 2). Examples of the polynucleotide include DNA and RNA, and DNA is preferable.

The present disclosure also relates to an expression vector containing the polynucleotide and a promoter operably linked thereto. Examples of the promoter include a promoter of a gene highly expressed in a desired host cell, and a virus-derived promoter. The promoter may be a constitutive promoter or an inducible promoter.

The expression vector may also further include elements such as terminators that function in the host cell, ribosome binding sites, and drug resistance genes. Examples of the drug resistance gene include resistance genes against drugs such as tetracycline, ampicillin, kanamycin, hygromycin, and phosphinothricin.

The expression vector may also further contain a region that allows for homologous recombination with the genome of the host cell for homologous recombination with the genomic DNA of the host cell. For example, the expression vector may be designed such that the expression unit contained therein is located between a pair of homologous regions (for example, homology arms, loxP, and FRT, homologous to specific sequences in the genome of the host cell). The genomic region (target of the homologous region) of the host cell into which the expression unit is to be introduced is not particularly limited, and may be a locus of a gene having a high expression level in the host cell.

The expression vector may be a plasmid, a viral vector, a phage, or an artificial chromosome. The expression vector may also be an integrative or non-integrative vector. An integrative vector may be a vector of a type that is entirely integrated into the genome of a host cell. Alternatively, an integrative vector may be a vector of a type in which only a portion (for example, expression units) thereof is integrated into the genome of a host cell. The expression vector may further be a DNA vector or an RNA vector (for example, a retrovirus).

The present disclosure also relates to a transformed cell that contains an expression unit containing the polynucleotide and a promoter operably linked thereto.

The "expression unit" refers to the smallest unit that contains a certain polynucleotide to be expressed as a protein and a promoter operably linked thereto, and allows transcription of the polynucleotide and the production of the protein encoded by the polynucleotide. The expression unit may further contain elements such as a terminator, a ribosome binding site, and a drug resistance gene. The expression unit may be DNA or RNA, and is preferably DNA. The expression unit may also be an expression unit that contains one polynucleotide to be expressed as a protein and a promoter operably linked thereto (that is, an expression unit allowing expression of monocistronic mRNA) or an expression unit that contains a plurality of polynucleotides to be expressed as a protein (for example, a polynucleotide encoding a heavy chain and a polynucleotide encoding a light chain) and a promoter operably linked thereto (that is, an expression unit allowing expression of polycistronic mRNA). The expression unit may be contained in the genomic region at one or more (for example, one, two, three, four, or five) different positions. Specific forms of the expression unit contained in the non-genomic region include plasmids, viral vectors, phages, and artificial chromosomes, for example. The promoter is the same as described above.

The transformed cell can be produced by any method known in the art. For example, the transformed cell can be produced by a method using an expression vector (for example, a competent cell method and an electroporation method) or a genome editing technique. If the expression vector is an integrative vector that results in homologous recombination with the genomic DNA of the host cell, the expression unit can be integrated into the genomic DNA of the host cell by transformation. Meanwhile, when the expression vector is a non-integrative vector that does not undergo homologous recombination with the genomic DNA of the host cell, the expression unit is not incorporated into the genomic DNA of the host cell by transformation, and can exist independently of the genomic DNA in the host cell in the state of the expression vector. Alternatively, according to a genome editing technology (for example, CRISPR/Cas system and Transcription Activator-Like Effector Nucleases (TALEN)), it is possible to incorporate an expression unit into the genomic DNA of a host cell. Examples of the host cell of the transformed cell include eukaryotic cells [for example, animal cells (for example, mammalian cells), plant cells, and eukaryotic microorganisms] and prokaryotic cells (for example, prokaryotic microorganisms).

### Examples

The present disclosure will be described in more detail with reference to the following examples, but the present disclosure is not limited to the following examples.

### Example 1: Preparation of human denatured CRP-specific monoclonal antibody

### (1) Preparation of antigen (human mCRP)

Human pentameric CRP (C-Reactive Protein Human Pleural Fluid, Lee biosolutions, catalogue number: 140-11) (human pCRP) was treated in 8 M Urea/10 mM EDTA at 37°C for 2 hours to prepare a human monomeric CRP (human mCRP) solution. After preparation, dialysis was performed in a 10 mM phosphate buffer (pH 7.4) and 15 mM NaCl using a dialysis tube. The dialyzed solution was transferred to a dialysis tube, then centrifuged at 3,500 xg at 4°C, and concentrated.

### (2) Immunization of mice with antigen (human mCRP)

The antigen (human mCRP) concentration was prepared to 200 µg/0.5 mL, and the same amount of adjuvant was added to prepare an emulsion using a glass syringe. Freund's complete adjuvant (FCA, DIFCO, USA) was used as an adjuvant only at the time of initial immunization, and Freund's incomplete adjuvant (FICA, DIFCO, USA) was used for the second and subsequent immunizations. After four times of immunizations, an increase in antibody titer was checked, and final immunization (i.p./50 µg/animal) was performed.

The obtained emulsion was injected subcutaneously and intradermally into mice using a 27G injection needle. Thereafter, a total of four times of immunizations were performed every 7 days, and a small amount of blood was collected from the tail vein 7 days after the four times of immunizations. A diluted antiserum was added to an immunoplate on which human mCRP was immobilized, and the titer of the antiserum was checked by anti-mouse IgG-HRP detection. Specifically, the titer of the antiserum was checked by the following solid phase ELISA.

### (3) Solid Phase ELISA

As materials for solid phase ELISA, the following were used.
(a) Plate: Immunoplate for ELISA (96 well) manufactured by Nunc
(b) Substrate: O.P.D tablet (SIGMA)
(c) Solid-phase buffer: 0.1 M carbonate buffer, pH 9.5 (IBL)
(d) Antiserum dilution buffer: 1% BSA, 0.05% Tween-20 (Kanto Chemical Co., Inc.) in PBS
(e) Labeled antibody dilution buffer: 1% BSA, 0.05% Tween-20 in PBS
(f) Washing buffer: 0.05% Tween-20 in 0.1 M phosphate buffer
(g) Substrate buffer: K₂HPO₄-citrate buffer (pH 5.1)
(h) Reaction stop solution: 1 mol/L-H₂SO₄ (Wako Pure Chemical Industries, Ltd.)
(i) Solid phase protein: human mCRP or BSA (control)

The solid phase ELISA was performed by the following procedure.
(a) The protein was immobilized on a plate by standing at 50 ng/well (50 µL/well) for 16 hours at 4°C.
(b) The solid phase protein was washed twice with Dulbecco's PBS (D-PBS) (200 µL/well).
(c) Blocking was performed by adding 200 µL of D-PBS containing 0.1% BSA and 0.05% NaN₃ to each well and leaving it at 4°C overnight.
(d) Each well was washed twice with the washing buffer.
(e) The sample (antiserum) was diluted with a dilution buffer, and the diluted solution was added to each well (50 µL/well) and left at 37°C for 30 minutes.
(f) Each well was washed four or more times with the washing buffer.
(g) Anti-mouse IgG (y-specific)-HRP, a labeled antibody, was added to each well (50 µL/well) and left at 37°C for 30 minutes.
(h) Each well was washed four or more times with the washing buffer.
(i) A chromogenic reaction was started by adding a substrate solution (400 µg/mL) to each well (100 µL/well), and the reaction was allowed to proceed for 15 minutes at room temperature in the dark.
(j) After 15 minutes, the reaction was stopped by addition of 1 mol/L sulfuric acid (100 µL/well), and absorbance (490 nm) was measured.

### (4) Preparation of hybridoma that produces human denatured CRP-specific antibody

Spleen cells or lymph node cells collected from a mouse in which a significant increase in titer to human mCRP was observed by the solid phase ELISA were cell-fused with myeloma cells (X-63.Ag8 653 (ATCC)) using polyethylene glycol (PEG) (19 pieces of 96 well plates). Hybridomas were selectively cultured using a HAT (hypoxanthine, aminopterin, and thymidine) selective medium. Thereafter, the colony formation of the hybridoma was observed, and screening by the above-described solid phase ELISA (solid phase antigen: human mCRP) was performed using a supernatant dilution of the culture of the hybridoma as a sample, and 36 positive clones were selected.

### (5) Inhibition ELISA

Next, hybridomas that produce human denatured CRP-specific antibodies having an ability of binding to human mCRP were selected by inhibition ELISA.

In the inhibition ELISA, a solid phase ELISA (solid phase antigen: human mCRP) as described above was performed using (i) a solution obtained by pre-incubating the culture supernatant of the hybridoma with a solution containing an excess amount of human mCRP, (ii) a solution obtained by pre-incubating the culture supernatant of the hybridoma with a solution containing an excess amount of human pCRP, and (iii) a solution (control) obtained by pre-incubating the culture supernatant of the hybridoma with a solution not containing both of human mCRP and human pCRP as samples. In such an inhibition ELISA, the culture supernatant of the hybridoma that produces the anti-human mCRP antibody having the ability of specifically binding to human mCRP does not bind to the solid phase antigen (human mCRP) because it binds to human mCRP in the solution in (i), and therefore a signal is not substantially detected. Meanwhile, in (ii), since such a culture supernatant does not bind to human pCRP in the solution and thus can bind to a solid phase antigen (human mCRP), a sufficient signal is detected. Further, in (iii), such a culture supernatant can bind to a solid phase antigen (human mCRP) because human mCRP and human pCRP are not present in the solution, and thus a sufficient signal is detected.

Specifically, the inhibition ELISA was performed as follows.
(a) Human mCRP was adjusted to 40 µg/mL with a dilution buffer (Solution a1). Alternatively, human pCRP was adjusted to 40 µg/mL with a dilution buffer (Solution a2) .
(b) The culture supernatant was diluted 2-fold with a dilution buffer (solution 2).
(c) Each 50 µL of Solution a1 and Solution 2 were mixed, and the antibody in the culture supernatant and human mCRP were reacted at 4°C for 16 hours or more. In addition, 50 µL each of Solution a2 and Solution 2 were mixed, and the antibody in the culture supernatant and human pCRP were reacted at 4°C for 16 hours or more.
(d) The subsequent procedures were similar to the procedures of (a) to (j) described above for the solid phase ELISA. The solution obtained in (c) of the inhibition ELISA was used as the sample used in (e) of the solid phase ELISA. The results are shown in Table 1.

### [Table 1]

**Table 1. Results of inhibition ELISA for 36 positive clones**

| | (i) mCRP | (ii) pCRP | (iii) control |
|---|---|---|---|
| 1 | 0.001 | 0.203 | 0.303 |
| 2 | 0.007 | -0.002 | 0.023 |
| 3 | 0.043 | 1.245 | 1.390 |
| 4 | 0.000 | -0.002 | 0.009 |
| 5 | 0.014 | 0.357 | 0.414 |
| 6 | 0.031 | 0.237 | 0.258 |
| 7 | 0.036 | 1.311 | 1.533 |
| 8 | 0.024 | 0.010 | 0.019 |
| 9 | 0.001 | 0.002 | 0.007 |
| 10 | 0.021 | 0.046 | 0.027 |
| 11 | 0.067 | 0.103 | 0.103 |
| 12 | 0.826 | 2.197 | 2.175 |
| 13 | 0.068 | 1.809 | 1.977 |
| 14 | 0.039 | 1.020 | 1.347 |
| 15 | 0.004 | 0.205 | 0.394 |
| 16 | 0.022 | 0.556 | 0.945 |
| 18 | 0.921 | 2.446 | 2.291 |
| 19 | 2.024 | 2.366 | 2.293 |
| 20 | 0.094 | 1.882 | 2.007 |
| 21 | 0.220 | 2.243 | 2.129 |
| 22 | -0.002 | 0.048 | 0.205 |
| 24 | -0.002 | -0.006 | 0.003 |
| 25 | 0.005 | 0.081 | 0.182 |
| 26 | 0.013 | 0.047 | 0.142 |
| 28 | 0.712 | 2.297 | 2.227 |
| 29 | 0.071 | 2.152 | 2.062 |
| 30 | 0.117 | 2.085 | 2.098 |
| 32 | 0.021 | 0.466 | 0.713 |
| 33 | -0.001 | 0.093 | 0.234 |
| 34 | 0.004 | 0.192 | 0.411 |
| 35 | 0.045 | 1.409 | 1.671 |
| 36 | 0.180 | 2.151 | 2.123 |

As a result, in many of the 36 positive clones, sample (i) showed a smaller signal value than samples (ii) and (iii) did. This indicates that many clones produce anti-human mCRP antibodies with the ability to specifically bind to human mCRP. Among these clones, seven kinds of clones (3, 12, 18, 19, 21, 35, and 36) in which signals were not substantially detected in (i) while sufficient signals were detected in (ii) and (iii) were selected. It is believed that these seven kinds of clones can produce excellent human denatured CRP-specific antibodies having the ability to bind to human mCRP.

### (6) Primary cloning of hybridomas and subclass identification

The selected seven kinds of clones were primary cloned by a limiting dilution method. After two weeks, screening was performed by an EIA method to select positive wells. The positive wells were selected for each 3 wells (A, B, and C), the supernatant was collected, and solid-phase ELISA and inhibition ELISA were performed. As a result, good antibody reactivity was not observed in one kind of clone, but good antibody reactivity was observed in three kinds of subclones (A, B, and C) corresponding to six kinds of clones (clone numbers 3, 12, 18, 19, 21, and 35). In the subsequent experiments, these subclones (3C, 12C, 18A, 19C, 21A, and 35A) were used.

Next, subclass check by sandwich ELISA was performed using one culture supernatant of each clone, and the results were as follows.
(a) mCRP-3C: mouse IgG2b, κ
(b) mCRP-12C: mouse IgG1, κ
(c) mCRP-18A: mouse IgG2a, κ
(d) mCRP-19C: mouse IgG1, κ
(e) mCRP-21A: mouse IgG2a, κ
(f) mCRP-35A: mouse IgG2a, κ

### (7) IgG purification

IgG was purified from the culture supernatant obtained by culturing six kinds of hybridomas (3C, 12C, 18A, 19C, 21A, and 35A) by a conventional method, and the binding ability to mCRP was analyzed.

Materials and instruments used for purification and analysis of IgG are as follows.
(a) Protein A column
(b) Binding buffer (glycine-NaCl, pH 8.9)
(c) Elution buffer (citric acid, pH 4.0)
(d) Regeneration buffer (citric acid, pH 2.0)
(e) D-PBS
(f) Absorbance monitor-280 nm (ATTO)
(g) AKTA System (GE Healthcare)
(h) Analytical column for AKTA (GE Healthcare)

Purification and analysis of IgG were performed as follows.

### (Purification of IgG)

(a) The culture supernatant was filtered at 0.45 µm, subjected to salting-out with ammonium sulfate, and then diluted with the binding buffer.
(b) The resultant was added to the Protein A column equilibrated with the binding buffer.
(c) The column was sufficiently washed with the binding buffer to remove contaminant proteins.
(d) The resultant is subjected to IgG elution with the elution buffer. A neutralization buffer in an amount of about 1/10 of the recovered liquid was previously placed in a recovery container to neutralize the pH of the eluate.
(e) The eluate was dialyzed under refrigeration in PBS.
(f) IgG was collected and filtered at 0.22 µm.
(g) The OD of 280 nm was measured to calculate the IgG concentration.
(h) The mixture was concentrated to about 5 mg/mL by an ultrafiltration method, filtered at 0.22 µm, and then dispensed and cryopreserved.

(Analysis of IgG)
(a) The absorbance at 280 nm was measured, and the protein concentration was calculated with A₂₈₀ = 1.382 as 1 mg/mL.
(b) The purity was checked by gel filtration analysis with AKTA FPLC.
(c) The titer was checked by the solid phase ELISA described above.

As a result, it was confirmed that all of the IgGs purified from the culture supernatant obtained by culturing the six kinds of hybridomas had binding ability to human mCRP.

### Example 2: Secondary screening of hybridomas capable of producing human denatured CRP-specific monoclonal antibodies

The subclones (mCRP-3C, mCRP-12C, and mCRP-18A) of the hybridoma obtained in Example 1 were secondarily cloned by a limiting dilution method. Cell culture and purification and analysis of IgG were performed in the same manner as in Example 1.

As a result, when the titer was checked by the solid phase ELISA described above, it was confirmed that all of the IgGs purified from the culture supernatant obtained by culturing the three kinds of hybridomas (mCRP-3C, mCRP-12C, and mCRP-18A) subjected to the secondary cloning have binding ability to human mCRP.

Further, when IgGs purified from the culture supernatant obtained by culturing these hybridomas were analyzed by the inhibition ELISA described in Example 1 (5), it was confirmed that these IgGs were human denatured CRP-specific monoclonal antibodies having the ability of binding to human mCRP (FIGS. 2 to 4).

### Example 3: Structural analysis of human denatured CRP-specific monoclonal antibody

mRNA was extracted from the three kinds of hybridomas (mCRP-3C, mCRP-12C, and mCRP-18A) secondarily screened in Example 2, and cDNA was prepared using a reverse transcriptase. A 5'-RACE (rapid amplification of cDNA ends) method was performed using primers specific to murine heavy chain and light chain to determine the N-terminal base sequences of the heavy chains and light chains. Full-length cDNAs of a heavy chain and a light chain were cloned into an expression vector using a translation initiation methionine peripheral sequence of an N-terminal base sequence as an N-terminal primer. The CDRs and variable regions were determined according to the definition of Kabat et al. utilizing IgBLAST from NCBI.

As a result, the human denatured CRP-specific monoclonal antibody produced by each hybridoma had the structural characteristics shown in Table 2 below (see also FIGS. 5 and 6).

### [Table 2]

**Table 2. Structures of human denatured CRP-specific monoclonal antibodies produced by various hybridomas**

| | Heavy chain | Light chain |
|---|---|---|
| mCRP-3C | Full-length nucleotide sequence: SEQ ID NO: 3 | Full-length nucleotide sequence: SEQ ID NO: 8 |
| | Full-length amino acid sequence: SEQ ID NO: 4 | Full-length amino acid sequence: SEQ ID NO: 9 |
| | CDR1: DYEMH (SEQ ID NO: 5) | CDR1: RSSQSLVHSNENTYLL (SEQ ID NO: 10) |
| | CDR2: AIHPGRGGTAYNQKFKG (SEQ ID NO: 6) | |
| | | CDR2: KVSNRFS (SEQ ID NO: 11) |
| | CDR3: YHGGY (SEQ ID NO: 7) | CDR3: SQTTHVPWT (SEQ ID NO: 12) |

From the above, it was shown that the antibody containing CDRs as described above can recognize human mCRP.

Hereinafter, the following abbreviations may be used for a human denatured CRP-specific antibody.
1) Human denatured CRP-specific antibody produced from hybridoma mCRP-3C: 3C antibody
2) Human denatured CRP-specific antibody produced from hybridoma mCRP-12C: 12C antibody
3) Human denatured CRP-specific antibody produced from hybridoma mCRP-18A: 18A antibody

### Example 4: Epitope analysis of human denatured CRP-specific monoclonal antibodies

### (1) Peptide synthesis (part 1)

Epitopes of 3C antibodies were analyzed. First, the following peptides used for epitope analysis were synthesized. Each of the following peptides has a cysteine residue (C) added to the C-terminus of the epitope of human mCRP in order to enable immobilization to a solid phase.
1) mCRP(19):QTDMSRKAFVC (SEQ ID NO: 13)
2) mCRP(29):FPKESDTSYVC (SEQ ID NO: 14)
3) mCRP(39):SLKAPLTKPLC (SEQ ID NO: 15)
4) mCRP(49):KAFTVCLHFYC (SEQ ID NO: 16)
5) mCRP(59):TELSSTRGYSC (SEQ ID NO: 17)
6) mCRP(69):IFSYATKRQDC (SEQ ID NO: 18)
7) mCRP(79):NEILIFWSKDC (SEQ ID NO: 19)
8) mCRP(89):IGYSFTVGGSC (SEQ ID NO: 20)
9) mCRP(99):EILFEVPEVTC (SEQ ID NO: 21)
10) mCRP(109):VAPVHICTSWC (SEQ ID NO: 22)
11) mCRP(119):ESASGIVEFWC (SEQ ID NO: 23)
12) mCRP(129):VDGKPRVRKSC (SEQ ID NO: 24)
13) mCRP(139):LKKGYTVGAEC (SEQ ID NO: 25)
14) mCRP(149):ASIILGQEQDC (SEQ ID NO: 26)
15) mCRP(159):SFGGNFEGSQC (SEQ ID NO: 27)
16) mCRP(169):SLVGDIGNVNC (SEQ ID NO: 28)
17) mCRP(179):MWDFVLSPDEC (SEQ ID NO: 29)
18) mCRP(189):INTIYLGGPFC (SEQ ID NO: 30)
19) mCRP(199):SPNVLNWRALC (SEQ ID NO: 31)
20) mCRP(209):KYEVQGEVFTKPQLWPC (SEQ ID NO: 32)

### (2) Epitope analysis (part 1)

The following materials were used as materials for epitope analysis.
(a) Plate: Immunoplate for ELISA (96 well) manufactured by Nunc
(b) Substrate: O.P.D tablet (SIGMA)
(c) Solid-phase buffer: 0.1 M carbonate buffer pH 9.5 (IBL)
(d) Antiserum dilution buffer: 1% BSA, 0.05% Tween-20 (Kanto Chemical Co., Inc.) in PBS
(e) Labeled antibody dilution buffer: 1% BSA, 0.05% Tween-20 in PBS
(f) Washing buffer: 0.05% Tween-20 in 0.1 M PB
(g) Substrate buffer: K₂HPO₄-citrate buffer (pH 5.1) (IBL)
(h) Reaction stop solution: 1 mol/L-H₂SO₄ (Wako Pure Chemical Industries, Ltd.)
(i) Solid phase peptide

Epitope analysis was performed by the following procedure.
(a) Peptides were immobilized on a plate by standing at 50 ng/well (50 µL/well) for 16 hours at 4°C.
(b) Solid phase peptides were washed twice with Dulbecco's PBS (D-PBS) (200 µL/well).
(c) Blocking was performed by adding 200 µL of D-PBS containing 0.1% BSA and 0.05% NaN₃ to each well and leaving it at 4°C overnight.
(d) Each well was washed twice with the washing buffer.
(e) The antibody was diluted to 1 µg/mL with the dilution buffer and the diluted solution was added to each well (50 µL/well) and left at 37°C for 30 minutes.
(f) Each well was washed four or more times with the washing buffer.
(g) Anti-mouse IgG (y-specific)-HRP, a labeled antibody, was added to each well (50 µL/well) and left at 37°C for 30 minutes.
(h) Each well was washed four or more times with the washing buffer.
(i) A chromogenic reaction was started by adding a substrate solution (400 µg/mL) to each well (100 µL/well), and the reaction was allowed to proceed for 15 minutes at room temperature in the dark.
(j) After 15 minutes, the reaction was stopped by addition of 1 mol/L sulfuric acid (100 µL/well), and absorbance (490 nm) was measured.

As a result, the 3C antibody exhibited reactivity to the peptide comprising EILFEVPEVT (SEQ ID NO: 2) similar to that of mCRP (Table 3). In the above (e), the same result was obtained even when the antibody concentration added to each well was changed to 5 µg/mL. When it was checked whether the 12C antibody and the 18A antibody (FIGS. 3 and 4), which are human denatured CRP-specific monoclonal antibodies, bind to a peptide comprising EILFEVPEVT (SEQ ID NO: 2) or not, they did not bind to the peptide. This indicates that these antibodies recognize an epitope different from that in the case of the 3C antibody. Accordingly, it is believed that there are not only one kind of epitope but also a plurality of kinds of epitopes of human denatured CRP-specific monoclonal antibodies.

### [Table 3]

**Table 3. Epitope analysis of 3C antibody (part 1)**

| | | | | | | | |
|---|---|---|---|---|---|---|---|
| | 19 | 29 | 39 | 49 | 59 | 69 | 79 |
| Absorbance | 0.004 | -0.001 | 0.001 | 0 | 0.001 | 0.002 | 0 |
| | | | | | | | |
| | 89 | 99 | 109 | 119 | 129 | 139 | 149 |
| Absorbance | 0 | 1.581 | -0.001 | 0.005 | 0.002 | 0.002 | 0.001 |
| | | | | | | | |
| | 159 | 169 | 179 | 189 | 199 | 209 | 159 |
| Absorbance | 0.002 | 0.001 | 0.003 | 0.007 | 0.006 | 0 | 0.002 |

From the above, it was considered that a plurality of epitopes comprising EILFEVPEVT (SEQ ID NO: 2) (FIG. 1), which is an epitope of the 3C antibody, exist as an epitope of the human denatured CRP-specific monoclonal antibody.

### (3) Peptide synthesis (part 2)

Epitopes of 3C antibodies were analyzed for replication. As the epitope, in place of the peptides of the above 1) to 20), the following longer peptides were synthesized and used.
21) mCRP(1-30):QTDMSRKAFVFPKESDTSYVSLKAPLTKPLC (SEQ ID NO: 33)
22) mCRP(31-60):KAFTVCLHFYTELSSTRGYSIFSYATKRQDC (SEQ ID NO: 34)
23) mCRP(61-90):NEILIFWSKDIGYSFTVGGSEILFEVPEVTC (SEQ ID NO: 35)
24) mCRP(91-120):VAPVHICTSWESASGIVEFWVDGKPRVRKSC (SEQ ID NO: 36)
25) mCRP(121-150):LKKGYTVGAEASIILGQEQDSFGGNFEGSQC (SEQ ID NO: 37)
26) mCRP(151-180):SLVGDIGNVNMWDFVLSPDEINTIYLGGPFC (SEQ ID NO: 38)
27) mCRP(181-206):SPNVLNWRALKYEVQGEVFTKPQLWPC (SEQ ID NO: 39)

### (4) Epitope analysis (part 2)

The material for epitope analysis was the same as that in Example 4 (2). The procedure for epitope analysis was the same as that in Example 4 (2).

As a result, the 3C antibody reacted to a peptide comprising EILFEVPEVT (SEQ ID NO: 2) (Table 4). The same results were also obtained when the peptide was not directly immobilized on the solid phase but immobilized on the solid phase via bovine serum albumin (BSA).

### [Table 4]

**Table 4. Epitope analysis of 3C antibody (part 2)**

| | | | | | | | |
|---|---|---|---|---|---|---|---|
| | 1-30 | 31-60 | 61-90 | 91-120 | 121-150 | 151-180 | 181-206 |
| Absorbance | -0.001 | 0.03 | **1.982** | 0.03 | 0.017 | 0.108 | 0.006 |

From the above, it was confirmed that the epitope of the 3C antibody was EILFEVPEVT (SEQ ID NO: 2) (FIG. 1).

### Example 5: Examination of effect of inhibiting mCRP binding ability by human denatured CRP-specific antibody

Binding of mCRP to the cell surface is mediated via Fc receptors and integrins. Accordingly, whether the produced antibody inhibits the binding of mCRP to the cell surface or not was examined. As blood cells, peripheral blood mononuclear cells (PBMCs) derived from a healthy subject were used, and binding of FAM-labeled mCRP to the cell surface was analyzed by a flow cytometer. Peripheral blood mononuclear cells derived from a healthy subject were separated from healthy subject's blood cells by ficoll (GE: Ficoll-Paque Premium). Binding between mCRP and PBMCs was checked by incubating FAM-mCRP and PBMCs for 5 minutes. For the examination of binding inhibition, 100 µg/ml of mCRP and 100 µg/ml of an anti-mCRP antibody (3C, 12C, 18A, 19C, 21A, or 35A) were pre-incubated for 15 minutes at room temperature (N = 3).

As a result, a part of the anti-mCRP antibodies (3C and 35A) strongly suppressed the binding of mCRP to PBMCs (FIG. 7). Accordingly, it was confirmed in vitro that the human denatured CRP-specific antibodies (3C and 35A) can strongly inhibit the binding ability of mCRP.

### Example 6: Examination of neutralizing action of anti-human denatured CRP antibody

In the absence of mCRP, peripheral blood mononuclear cells cannot bind to fibrinogen, but in the presence of mCRP, integrin is activated, so that fibrinogen can bind to the cell surface. It was examined whether the anti-mCRP antibody has a neutralizing action of suppressing the effect of mCRP.

As blood cells, peripheral blood mononuclear cells derived from a healthy subject were used, and binding of FAM-labeled fibrinogen (yCtrunc399) to the cell surface was analyzed by a flow cytometer. Peripheral blood mononuclear cells derived from a healthy subject were separated from patient's blood cells by ficoll (GE: Ficoll-Paque Premium). Integrin activation was induced by stimulating mononuclear cells with 100 µg/ml of mCRP. For the examination of the neutralizing action, 100 µg/ml of mCRP and 100 µg/ml of an anti-mCRP antibody (3C, 12C, 18A, 19C, 21A, or 35A) were pre-incubated for 15 minutes at room temperature (N = 3).

As a result, some of human denatured CRP-specific antibodies (3C and 35A) showed a neutralizing action that strongly suppresses integrin activation by mCRP (FIGS. 8A to 8D). Accordingly, it was confirmed in vitro that human denatured CRP-specific antibodies (3C and 35A) are neutralizing antibodies having a strong neutralizing action on mCRP. Further, it was confirmed that EILFEVPEVT (SEQ ID NO: 2) that is an epitope recognized by the human denatured CRP-specific antibody (3C) is an excellent neutralizing epitope.

### Example 7: Examination of usefulness of neutralizing antibody specific to human denatured CRP using peritonitis model mouse

Intraperitoneal administration of thioglycolate stimulates migration of inflammatory cells into the abdominal cavity. Normally, migration of neutrophils reaches the peak after 24 hours, and migration of monocytes reaches the peak after 48 hours. Using the peritonitis model, by counting the number of inflammatory cells in the abdominal cavity after administration of thioglycolate, the degree of inflammatory reaction (peritonitis) can be easily grasped. Using this model, whether a neutralizing antibody specific to human denatured CRP suppresses thioglycolate-induced peritonitis or not was examined.

Peritonitis was induced in 7-week-old BALB/C mice (SHIMIZU Laboratory Supplies Co., Ltd.) by intraperitoneal administration of 1 ml of a 4% thioglycolate solution. Further, a neutralizing antibody (3C or 35A) specific to human denatured CRP or a control IgG antibody was administered at 80 µg/body, and human denatured CRP was administered at 50 µg/body. After 48 hours, the mice were euthanized (central disruption), 10 ml of PBS was injected into the abdominal cavity, and ascites was collected. 1 ml of the collected ascites was dispensed and reacted with anti-mouse Gr-1-PE (Biolegend RB6-8C5) and rat anti-mouse F4/F80-FITC (abcam BM8), and GR-1 positive cells (neutrophils) and F4/F80 positive cells (monocytes/macrophages) were counted by a flow cytometer.

As a result, the neutralizing antibody specific to human denatured CRP suppressed thioglycolate-induced peritonitis (FIG. 9). Accordingly, it was confirmed that a neutralizing antibody specific to human denatured CRP can be used as a therapeutic agent for inflammatory diseases.

### Example 8: Examination of usefulness of neutralizing antibody specific to human denatured CRP using arthritis model mouse (preventive effect)

Since collagen-induced arthritis (CIA) is induced by an autoantibody against type II collagen, arthritis can be developed in a mouse by administration of an anti-type II collagen monoclonal antibody and a lipopolysaccharide (LPS).

Using 7-week-old DBA/1J mice (SHIMIZU Laboratory Supplies Co., Ltd.), 1.25 mg of a cocktail of five kinds of monoclonal antibodies (Arthrogen-CIA: Arthritogenic Monoclonal Antibody, Chondrex, USA) against type II collagen was administered intraperitoneally on Day 0 (N = 10). On Day 3, LPS (25 µg) (N = 10) and a neutralizing antibody 3C specific to human denatured CRP (100 µg) (N = 5) or control IgG (100 µg) (N = 5) were intraperitoneally administered. Joint scores were evaluated by scoring of 4 points for one limb with the maximum point of 16 points. The scores were evaluated for three joints: finger, top of hand, and wrist (0: no change; 1: swelling of any one joint; 2: swelling of any two joints; 3: swelling of all joints; 4: swelling of all joints and swelling of the entire limb in red). The measurement was performed every 2 to 3 days from DAY 0 to DAY 14.

Arthritis was induced from Day 4 and reached a maximum value on Days 10 to 15. In the group to which the 3C antibody was administered, a decrease in joint score was observed as compared with the group to which the IgG antibody was administered (FIG. 10A). Mild infiltration of inflammatory cells was observed in the 3C administration group, whereas marked infiltration of inflammatory cells, pannus formation, and destruction of articular cartilage were observed in the IgG administration group (FIG. 10B). Accordingly, it was confirmed that a neutralizing antibody specific to human denatured CRP can be used as a preventive agent for rheumatoid arthritis.

### Example 9: Examination of usefulness of neutralizing antibody specific to human denatured CRP using arthritis model mouse (therapeutic effect)

Using 7-week-old DBA/1J mice (SHIMIZU Laboratory Supplies Co., Ltd.), 1.25 mg of a cocktail of five kinds of monoclonal antibodies (Arthrogen-CIA: Arthritogenic Monoclonal Antibody, Chondrex, USA) against type II collagen was administered intraperitoneally on Day 0 (N = 10). LPS (25 µg) was administered intraperitoneally on Day 3 (N = 10). On Day 7, a neutralizing antibody 3C (100 µg) (N = 5) specific to human denatured CRP or control IgG (100 µg) (N = 5) was intraperitoneally administered. Arthritis was induced from Day 4 and reached a maximum value on Days 10 to 15. Joint scores were evaluated by scoring of 4 points for one limb with the maximum point of 16 points. The scores were evaluated for 3 joints: finger, top of hand, and wrist (0: no change; 1: swelling of any one joint; 2: swelling of any two joints; 3: swelling of all joints; 4: swelling of all joints and swelling of the entire limb in red). The measurement was performed every 2 to 3 days from DAY 0 to DAY 17.

As a result, in the group to which the 3C antibody was administered, a decrease in joint score was observed as compared with the group to which the IgG antibody was administered (FIG. 11A). In addition, an almost normal bone tissue was observed in the 3C administration group, whereas infiltration of inflammatory cells, pannus formation, and destruction of articular cartilage were observed in the IgG administration group (FIG. 11B). Accordingly, it was confirmed that a neutralizing antibody specific to human denatured CRP can be used as a therapeutic agent for rheumatoid arthritis.

### Example 10: Examination of usefulness of neutralizing antibody specific to human denatured CRP using systemic lupus erythematosus (SLE) model mouse

MRL/lpr mice exhibit an SLE phenotype from week 12 (lymph node swelling, nephritis, arthritis, and sialadenitis).

From week 11, a neutralizing antibody (3C) specific to human denatured CRP and a control IgG antibody were intraperitoneally administered to 9-week-old MRL/lpr mice (SHIMIZU Laboratory Supplies Co., Ltd.) (once a week; 100 µg/body) (control group: N = 6, and group 3C: N = 7) until week 18. During this time, urine protein was measured once a week. MYURIACE T manufactured by Terumo Corporation was used for urine protein measurement. Urine protein assessment was quantified by Tes-tape color change (0: -; ±: 15 mg/dl; +: 30 mg/dl; ++: 100 mg/dl; +++: 250 mg/dl). Mice were euthanized at week 19 (central disruption) and anti-double-stranded (ds)-DNA antibody titers were measured (cardiac puncture) (group receiving neutralizing antibody specific to human denatured CRP administration group: N = 7, and IgG administration group: N = 6). The anti-ds-DNA antibody titer was measured using an ELISA kit (LBIS-anti-ds-DNA-mouse ELISA kit) manufactured by FUJIFILM Corporation.

As a result, a decrease in anti-ds-DNA antibody titer and a decrease in urine protein were observed in the group to which the 3C antibody was administered, as compared with the group to which the IgG antibody was administered (FIGS. 12A and 12B). Accordingly, it was confirmed that a neutralizing antibody specific to human denatured CRP can be used as a therapeutic agent for SLE.

### Example 11: Preparation and analysis of humanized antibodies and chimeric antibody

### (1) Preparation of humanized antibodies and chimeric antibody

Humanized antibodies and a chimeric antibody that share the complementarity determining regions (CDRs) 1 to 3 in the heavy chain (HC) and light chain (LC) of the human denatured CRP-specific monoclonal antibody (3C antibody) obtained in the previous Examples were prepared, and their binding capacities were analyzed.

First, from the amino acid sequences of the heavy chain and the light chain of the 3C antibody, CDRs 1 to 3 were identified based on the Kabat and IMGT numbering schemes. The identified CDRs 1 to 3 were grafted into variable regions based on the human immunoglobulin heavy chain variable gene (IGHV) and the human immunoglobulin kappa variable gene (IGKV).

Next, the heavy chain variable region (VH) and the light chain variable region (VL) after transplantation were cloned into expression vectors having the amino acid sequences of the constant regions of IgG1 (G1m17,1) and IgK1 (Km3), respectively, to produce humanized antibodies containing a heavy chain (HCs 1 to 5) obtained by transplanting CDRs 1 to 3 in the heavy chain of the 3C antibody (mouse antibody) into a human antibody and a light chain (LCs 1 to 3) obtained by transplanting CDRs 1 to 3 in the light chain of the 3C antibody into a human antibody.

A chimeric antibody containing a heavy chain (HC 0) in which a heavy chain variable region of a 3C antibody (mouse antibody) was linked to a heavy chain constant region of a human antibody and a light chain (LC 0) in which a light chain variable region of a 3C antibody was linked to a light chain constant region of a human antibody was also prepared.

Information on the heavy chain (HCs 0 to 5) and light chain (LCs 0 to 3) of each of the produced antibodies is shown in Table 5.

### [Table 5]

**Table 5. Relationship between heavy chain and light chain of chimeric antibody and humanized antibody, and specific region**

| | SEQ ID NO | | Position of specific region in amino acid sequence | | | |
|---|---|---|---|---|---|---|
| | Nucleotide sequence | Amino acid sequence | Variable region | CDR1 | CDR2 | CDR3 |
| HC0 | SEQ ID NO: 40 | SEQ ID NO: 41 | Positions 20-133 | Positions 45-54 | Positions 69-85 | Positions 116-122 |
| HC1 | SEQ ID NO: 42 | SEQ ID NO: 43 | Positions 20-133 | Positions 45-54 | Positions 69-85 | Positions 116-122 |
| HC2 | SEQ ID NO: 44 | SEQ ID NO: 45 | Positions 20-133 | Positions 45-54 | Positions 69-85 | Positions 116-122 |
| HC3 | SEQ ID NO: 46 | SEQ ID NO: 47 | Positions 20-133 | Positions 45-54 | Positions 69-85 | Positions 116-122 |
| HC4 | SEQ ID NO: 48 | SEQ ID NO: 49 | Positions 20-133 | Positions 45-54 | Positions 69-85 | Positions 116-122 |
| HC5 | SEQ ID NO: 50 | SEQ ID NO: 51 | Positions 20-133 | Positions 45-54 | Positions 69-85 | Positions 116-122 |
| LC0 | SEQ ID NO: 52 | SEQ ID NO: 53 | Positions 21-132 | Positions 44-59 | Positions 75-81 | Positions 114-122 |
| LC1 | SEQ ID NO: 54 | SEQ ID NO: 55 | Positions 21-132 | Positions 44-59 | Positions 75-81 | Positions 114-122 |
| LC2 | SEQ ID NO: 56 | SEQ ID NO: 57 | Positions 21-132 | Positions 44-59 | Positions 75-81 | Positions 114-122 |
| LC3 | SEQ ID NO: 58 | SEQ ID NO: 59 | Positions 21-132 | Positions 44-59 | Positions 75-81 | Positions 114-122 |

### (2) Analysis of humanized antibodies and chimeric antibody

The binding ability of each of the prepared antibodies was analyzed. The expression vector was introduced into CHO cells and cultured, and then the expressed antibody was purified from the culture supernatant using an AKTA^{™} design chromatography system. The binding ability of the obtained 10 kinds of purified antibodies was analyzed by two kinds of methods.

First, the binding ability of the purified antibody was analyzed using Octet Systems utilizing Bio-Layer Interferometry (BLI). The binding ability of the humanized antibody (HCx/LCx) was evaluated in percentage with the binding ability of the chimeric antibody (HC 0/LC 0) as 100%.

Next, the binding ability of the purified antibody was analyzed by Biacore^{™}.

As a result, it was confirmed that all of the 10 kinds of purified antibodies exhibited a high binding ability (Table 6).

### [Table 6]

**Table 6. Binding ability of purified antibodies**

| | HC/LC* | Analysis of binding ability | | | | |
|---|---|---|---|---|---|---|
| | | BLI | Biacore^{™} | | | |
| | | | ka (M⁻¹s⁻¹) global fit | kd (s⁻¹) separate fit | K_{D} (M) | % of Rₘₐₓ theor |
| Chimeric antibody | HC0/LC0 | 100 | 3.78 E +03 | < 4.19 E-06 | < 1.11 E-09 | 379 |
| Humanized antibody 1 | HC1/LC3 | 132.13 | 3.88 E +03 | < 4.19 E-06 | < 1.08 E-09 | 417 |
| Humanized antibody 2 | HC2/LC1 | 100.69 | 2.97 E +03 | 7.58 E-06 | 2.55 E-09 | 235 |
| Humanized antibody 3 | HC2/LC3 | 109.05 | 3.04 E +03 | < 4.19 E-06 | < 1.38 E-09 | 226 |
| Humanized antibody 4 | HC3/LC1 | 96.42 | 3.77 E +03 | < 4.19 E-06 | < 1.11 E-09 | 353 |
| Humanized antibody 5 | HC3/LC3 | 100.93 | 3.68 E +03 | < 4.19 E-06 | < 1.14 E-09 | 400 |
| Humanized antibody 6 | HC4/LC1 | 109.67 | 2.79 E +03 | < 4.19 E-06 | < 1.50 E-09 | 309 |
| Humanized antibody 7 | HC4/LC2 | 79.71 | 2.27 E +03 | < 4.19 E-06 | < 1.84 E-09 | 228 |
| Humanized antibody 8 | HC4/LC3 | 92.08 | 2.92 E +03 | < 4.19 E-06 | < 1.44 E-09 | 375 |
| Humanized antibody 9 | HC5/LC1 | 92.87 | 3.39 E +03 | < 4.19 E-06 | < 1.24 E-09 | 306 |
| Humanized antibody 10 | HC5/LC3 | 116.93 | 3.69 E +03 | < 4.19 E-06 | < 1.14 E-09 | 392 |

| | | | | | | |
|---|---|---|---|---|---|---|
| * The combination of heavy chain (HC) and (light chain) in an antibody is shown. | | | | | | |

According to at least one embodiment described above, the neutralizing antibody can be used as a medicine such as an anti-inflammatory agent.

Although some embodiments of the present invention have been described, these embodiments have been presented as examples, and are not intended to limit the scope of the invention. These novel embodiments can be implemented in various other forms, and various omissions, substitutions, and changes can be made without departing from the gist of the invention. These embodiments and modifications thereof are included in the scope and gist of the invention, and are included in the invention described in the claims and the equivalent scope thereof.

[Sequence Listing]

## Claims

1. An anti-inflammatory agent comprising a neutralizing antibody specific to human denatured CRP.

2. The anti-inflammatory agent according to claim 1, wherein the neutralizing antibody has an ability of binding to EILFEVPEVT (SEQ ID NO: 2).

3. The anti-inflammatory agent according to claim 1 or 2, wherein the neutralizing antibody comprises the following 1) and 2) :
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.

4. The anti-inflammatory agent according to any one of claims 1 to 3, wherein the neutralizing antibody is IgG.

5. The anti-inflammatory agent according to any one of claims 1 to 4, wherein the anti-inflammatory agent is for inflammation resulting from an autoimmune disease.

6. The anti-inflammatory agent according to claim 5, wherein the autoimmune disease is rheumatoid arthritis or systemic lupus erythematosus.

7. A neutralizing antibody specific to human denatured CRP and having an ability of binding to EILFEVPEVT (SEQ ID NO: 2).

8. The neutralizing antibody according to claim 7, comprising the following 1) and 2):
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.

9. A polynucleotide comprising a nucleotide sequence encoding the neutralizing antibody according to claim 7 or 8.

10. An expression vector comprising the polynucleotide according to claim 9 and a promoter operably linked thereto.

11. A transformed cell comprising an expression unit containing the polynucleotide according to claim 9 and a promoter operably linked thereto.

12. A medicine comprising a neutralizing antibody specific to human denatured CRP and having an ability of binding to EILFEVPEVT (SEQ ID NO: 2).

13. The medicine according to claim 12, wherein the neutralizing antibody comprises the following 1) and 2):
1) an antibody heavy chain containing a variable region comprising CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7; and
2) an antibody light chain containing a variable region comprising CDR1 consisting of the amino acid sequence of SEQ ID NO: 10, CDR2 consisting of the amino acid sequence of SEQ ID NO: 11, and CDR3 consisting of the amino acid sequence of SEQ ID NO: 12.
